(19) Europäisches Patentamt
European Patent Office
Office européen des brevets

(11) Veröffentlichungsnummer: **0 007 520**
**B1**

(12) # EUROPÄISCHE PATENTSCHRIFT

(21) Anmeldenummer: **79102376.5**

(22) Anmeldetag: **11.07.79**

(51) Int. Cl.[3]: **C 07 D 265/30**

(54) **Verfahren zur Herstellung von cis-2,6-dimethylmorpholin**

(30) Priorität: **14.07.78 DE 2830998**

(43) Veröffentlichungstag der Anmeldung:
**06.02.80 Patentblatt 80/03**

(45) Bekanntmachung des Hinweises auf die Patenterteilung:
**07.01.81 Patentblatt 81/01**

(84) Benannte Vertragsstaaten:
**AT BE CH DE FR GB IT LU NL SE**

(56) Entgegenhaltungen:
**US - A - 3 083 202**

(73) Patentinhaber: **BASF Aktiengesellschaft**
**Carl-Bosch-Strasse 38**
**D - 6700 Ludwigshafen (DE)**

(72) Erfinder: **Goetz, Norbert, Dr. Chem.**
**Schoefferstrasse 25**
**D - 6520 Worms (DE)**
**Himmele, Walter, Dr. Chem.**
**Eichenweg 14**
**D - 6909 Walldorf (DE)**
**Hupfer, Leopold, Dr. Chem.**
**Waltershoehe 3**
**D - 6701 Friedelsheim (DE)**

**EP 0 007 520 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zur Herstellung von cis-2,6-dimethylmorpholin

Die vorliegende Erfindung betrifft ein Verfahren zur Herstellung von cis-2,6-Dimethylmorpholin aus trans-2,6-Dimethylmorpholin durch Isomerisierung an Katalysatoren in Gegenwart von Wasserstoff.

Es ist bekannt, daß man in einem Gemisch aus cis- und trans-2,6-Dimethylmorpholin durch Erhitzen dieses Gemisches mit konzentrierter oder rauchender Schwefelsäure bei Temperaturen zwischen 185 und 220°C den Anteil der cis-Verbindung erhöhen kann (US-Patentschrift 3 083 202). Weiterhin ist bekannt, daß sich in manchen Fällen cis/trans-Umlagerungen an Hydrierkatalysatoren durchführen lassen (Houben-Weyl, "Methoden der organischen Chemie", Band 4/2, Seiten 227—283). Beispielsweise läßt sich cis-1,4-Dimethylcyclohexan in Gegenwart eines Nickelkatalysators bei 175°C in trans-1,4-Dimethylcyclohexan umlagern [N. D. Zelinsky, E. J. Margolis, Ber. dtsch. chem. Ges. *65*, 1613 (1932)].

Es wurde nun gefunden, daß man cis-2,6-Dimethylmorpholin der Formel I

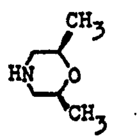

I

in einfacher Weise erhält, wenn man trans-2,6-Dimethylmorpholin der Formel II

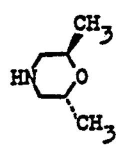

II

in Gegenwart von Wasserstoff und in Gegenwart eines Katalysators umsetzt, der ein oder mehrere Metalle aus der Gruppe VIII oder der Gruppe Ib des Periodensystems der Elemente oder deren Mischungen enthält.

Der Vorteil des erfindungsgemäßen Verfahrens besteht darin, daß man cis-2,6-Dimethylmorpholin weitgehend frei von Neben- und Abfallprodukten erhält. Im Vergleich hierzu führt die bekannte Isomerisierung mit Schwefelsäure bei der anschließenden Neutralisation der Mischung mit Alkalien und Freisetzung der 2,6-Dimethylmorpholinbase zu umweltbelastenden Stoffen in Form von unerwünschten Alkalisulfaten, die den Flüssen zugeleitet werden müssen.

Cis/trans-Umlagerungen mit entsprechend substituierten Morpholinringen in Gegenwart von Hydrierkatalysatoren sind bisher nicht bekannt.

Die erfindungsgemäße Umsetzung kann sowohl kontinuierlich als auch diskontinuierlich durchgeführt werden. In der Regel werden Temperaturen zwischen 120 und 280°C, vorteilhaft zwischen 150 und 250°C und Drucke zwischen 1 und 200 bar, vorteilhaft zwischen 1 und 50 bar angewendet. Es kann lösungsmittelfrei oder in Gegenwart von Lösungsmitteln, die unter den Reaktionsbedingungen inert sind, gearbeitet werden.

Als Lösungsmittel kommen beispielsweise in Frage: Cyclohexan, Cyclopentan, Methylcyclopentan, Hexan, Heptan, Cyclohexylmethyläther, Di-n-butyläther, Tetrahydrofuran, Dioxan.

Als Katalysatoren finden Nickel-, Kobalt-, Kupfer-, Silber- und insbesondere Palladium-Katalysatoren Verwendung. Die Katalysatoren können in reiner Form, d.h. nur ein Metall oder in Form von Mischkatalysatoren mit oder ohne Trägerstoffe eingesetzt werden. Als inerte Trägerstoffe eignen sich beispielsweise Aktivkohle, $SiO_2$ oder $Al_2O_3$. Weiterhin wirkt sich ein Zusatz von basischen Metalloxiden, beispielsweise von Oxiden aus der Gruppe der seltenen Erdmetalle, z.B. $Pr_2O_3$, $La_2O_3$, $CeO_2$, $Nd_2O_3$, $Gd_2O_3$ oder $Sm_2O_3$ sehr vorteilhaft aus. Der Katalysator enthält beispielsweise Zusätze zwischen 0,2 und 20 Gew.% eines Oxides oder eines Gemisches von Oxiden der Seltenen Erdmetalle.

Der Katalysator enthält beispielsweise 0,1 bis 98 Gew.% Metall. Der Katalysator kann auch noch weitere Stoffe z.B. Mangan oder Phosphorsäure enthalten.

Im allgemeinen werden die Katalysatoren in Form von Tabletten mit vorzugsweise 4 bis 5 mm Durchmesser oder in Form von Pulver verwendet.

Das nach dem Verfahren der Erfindung hergestellte cis-2,6-Dimethylmorpholin findet als Zwischenprodukt für die Herstellung von fungiziden Wirkstoffen für Pflanzenschutzmittel Verwendung (DE-OS 2 656 747).

Die in den folgenden Beispielen genannten Teile bedeuten Gewichtsteile; Raumteile verhalten sich zu ihnen wie Liter zu Kilogramm.

Beispiel 1

In einem Rollautoklaven mit einem Inhalt von 3000 Raumteilen werden 1500 Gewichtsteile trans-2,6-Dimethylmorpholin eingefüllt. Als Katalysator werden 5 Gewichtsteile Palladium-Aktivkohle mit 10% Palladiumgehalt zugegeben. Der Autoklav wird druckdicht verschlossen und zunächst mit Stickstoff gespült. Anschließend werden 10 bar Wasserstoff aufgepreßt und wieder entspannt. Der Autoklav wird dann auf 175°C aufgeheizt und 36 Stunden lang bei dieser Temperatur gehalten. Eine nach dieser Reaktionszeit entnommene Probe des Reaktionsgemisches zeigt bei der gaschromatographischen Analyse einen Gehalt von 53,5% cis-2,6-Dimethylmorpholin und 46,3% trans-2,6-Dimethylmorpholin. Nach

weiteren 36 Stunden Reaktionszeit bei 175°C steigt der Anteil des cis-Produktes auf 70,6% an; der trans-Anteil sinkt auf 29,2% ab.

Die Fraktionierung des Reaktionsgemisches über eine Destillier-Kolonne mit ca. 60 theoretischen Böden liefert 810 Teile reines cis-2,6-Dimethylmorpholin, $Kp_{100} = 80 - 81$°C. Das entspricht einer Ausbeute von 54% (ohne Berücksichtigung des rückführbaren Anteils an trans-2,6-Dimethylmorpholin). Weiterhin fallen 531 Teile einer Fraktion an, die überwiegend aus trans-2,6-Dimethylmorpholin besteht, $Kp_{100} = 87$—90°C. An höhersiedenden Produkten (Destillationsrückstand) werden 158 Teile erhalten. Die Selektivität der Reaktion beträgt 89,5%.

Beispiel 2

Wie in Beispiel 1 beschrieben, werden 1500 Teile trans-2,6-Dimethylmorpholin in Gegenwart von 5 Teilen des in Beispiel 1 beschriebenen Katalysators in Gegenwart von Wasserstoff 24 Stunden bei 200°C behandelt. Die gaschromatographische Analyse zeigt nach dieser Reaktionsdauer einen Anteil von 80% cis- und 20% trans-Verbindung. Die Fraktionierung des Reaktionsproduktes führt hier zu 965 Teilen reinem cis-2,6-Dimethylmorpholin. Das entspricht einer Ausbeute von 64,5% (ohne Berücksichtigung des zurückgewinnbaren Ausgangsproduktes trans-2,6-Dimethylmorpholin). An trans-2,6-Dimethylmorpholin werden 408 Teile gewonnen. Der Destillationsrückstand beträgt 126 Teile. Es wird eine Selektivität von 91,5% erhalten.

Beispiel 3

1500 Teile trans-2,6-Dimethylmorpholin werden in Gegenwart von 2 Teilen des unter Beispiel 1 näher beschriebenen Katalysators bei 225°C umgesetzt. Ansonsten wurden die unter Beispiel 1 näher beschriebenen Reaktionsbedingungen angewendet.

Nach jeweils 6 Stunden Reaktionszeit wird eine Probe von ca. 150 Teilen Reaktionsprodukt entnommen. Von dieser Probe wird die Zusammensetzung gaschromatographisch analysiert und eine Bestimmung des Destillationsrückstandes vorgenommen. Hierzu wurden die bis zu einer Temperatur von 205°C destillierbaren Produkte destilliert. Die Zusammensetzung des Destillats wird wiederum gaschromatographisch bestimmt. Die erhaltenen Analysenwerte sind in der folgenden Tabelle zusammengestellt:

| Reaktionszeit in Stunden | Anteil an cis-Verbindung im Rohaustrag | Anteil an trans-Verbindung im Rohaustrag | Anteil an cis-Verbindung im Destillat | Anteil an trans-Verbindung im Destillat | Gew. % des Dest.-Rückstandes |
|---|---|---|---|---|---|
| 6 | 31,4 % | 68,2 % | 30,1 % | 68,7 % | 8,5 % |
| 12 | 51,8 % | 48,1 % | 51,3 % | 47,1 % | 10,4 % |
| 18 | 63,0 % | 27,7 % | 61,5 % | 35,1 % | 12,1 % |
| 24 | 76,3 % | 22,6 % | 74,3 % | 25,1 % | 15,2 % |

Beispiel 4

Ein Rollautoklav mit einem Volumen von 1000 Raumteilen wird mit einer Mischung aus 100 Teilen trans-2,6-Dimethylmorpholin, 500 Teilen Tetrahydrofuran und 25 Teilen eines Katalysators gefüllt, der aus 7,9 Gew.% Nickel, 7,9 Gew.% Kobalt und 3,2 Gew.% Kupfer auf Aluminiumoxid besteht. Nach einer Spülung mit Stickstoff wird Wasserstoff bis zum Erreichen eines Druckes von 10 bar aufgepreßt. Dann wird der Autoklav auf 170°C erhitzt und 10 Stunden lang auf dieser Temperatur gehalten. Anschließend wird abgekühlt, der Katalysator abfiltriert und das Filtrat destillativ gereinigt. Hierbei werden 91 Teile eines Gemisches erhalten, das aus 89% 2,6-Dimethylmorpholin, mit der Zusammensetzung 51% cis- und 49% trans-Verbindung, besteht. Der Destillationsrückstand beträgt 9 Teile. Hieraus errechnet sich eine Selektivität von 81%.

Beispiel 5

In ein zylindrisches Reaktionsrohr mit einem Volumen von 500 Raumteilen wird ein Katalysator mit der Zusammensetzung von 91 Gew.% Kobalt, 5 Gew.% Mangan und 4 Gew.% $H_3PO_4$ in Form von Strängen (5 mm Durchmesser und 10 mm Länge) eingefüllt und auf 170°C erhitzt. Über dieses Katalysatorbett werden stündlich 60 Teile trans-2,6-Dimethylmorpholin geführt. Gleichzeitig werden 10 000 Raumteile Wasserstoff bei einem Druck von 50 bar im Gleichstrom hindurchgeleitet. Das aus dem Reaktionsrohr herausfließende Reaktionsprodukt wird unter Druck abgekühlt und dann entspannt. Es fallen hierbei stündlich 60 Teile Rohprodukt an, die destillativ gereinigt werden. Eine Probedestillation von 100 Teilen des Rohproduktes, die entsprechend der unter Beispiel 3 angegebenen Probedestillation durchgeführt wird, liefert folgende Ergebnisse:

Im Destillat (98 Teile) befinden sich
58,5% cis-2,6-Dimethylmorpholin,
31,5% trans-2,6-Dimethylmorpholin
Der Destillationsrückstand beträgt 1,5%.
Hieraus ergibt sich eine Selektivität von 88%.

Beispiel 6

In der unter Beispiel 5 näher beschriebenen Apparatur wird ein Katalysator mit der Zusammensetzung 0,36 Gew.% Palladium, 4,8 Gew.% Silber und 1 Gew.% Mangan auf $SiO_2$ in Form von Strängen (5 mm Durchmesser und 10 mm Länge) eingefüllt und auf 250°C erhitzt. Über diesen Katalysator werden stündlich 60 Teile trans-2,6-Dimethylmorpholin geführt. Gleichzeitig werden 10 000 Raumteile Wasserstoff bei einem Druck von 50 bar im Gleichstrom hindurchgeleitet. Das aus dem Reaktionsrohr herausfließende Reaktionsprodukt wird unter Druck abgekühlt und dann entspannt. Es fallen stündlich 60 Teile Rohprodukt an, die destillativ gereinigt werden.

Eine Probedestillation von 100 Teilen des Rohproduktes, die entsprechend den unter Beispiel 3 angegebenen Bedingungen ausgeführt wird, liefert folgende Ergebnisse:

Im Destillat befinden sich
32% cis-2,6-Dimethylmorpholin
68% trans-2,6-Dimethylmorpholin
Der Destillationsrückstand beträgt 11,5%.
Aus diesen Daten errechnet sich eine Selektivität von 88%.

Beispiel 7

In der unter Beispiel 5 näher beschriebenen Apparatur wird ein Katalysator, bestehend aus 0,5 Gew.% Palladium und 5 Gew.% Praseodymoxid, $Pr_2O_3$, auf Aluminiumoxid als Trägermaterial in Form von Strängen (5 mm Durchmesser und 10 mm Länge) eingefüllt und auf 230°C erhitzt. Über diesen Katalysator werden stündlich 60 Teile trans-2,6-Dimethylmorpholin und gleichzeitig 10 000 Raumteile Wasserstoff bei einem Druck von 50 bar geführt. Das aus dem Reaktionsrohr herausfließende Produkt wird unter Druck abgekühlt und entspannt. Es fallen stündlich 60 Teile Rohprodukt an, die destillativ gereinigt werden. Die Probedestillation von 100 Teilen des Rohproduktes liefert hier folgende Ergebnisse:

Im Destillat (97 Teile) befinden sich
73% cis-2,6-Dimethylmorpholin
24% trans-2,6-Dimethylmorpholin.
Der Destillationsrückstand beträgt 2,5%.
Er errechnet sich eine Selektivität von 94%.

Beispiel 8

Mit der unter Beispiel 5 beschriebenen Apparatur und dem unter Beispiel 7 beschriebenen Katalysator wird die Isomerisierung von trans-2,6-Dimethylmorpholin bei 250°C durchgeführt. Alle anderen Bedingungen sind mit denen von Beispiel 7 identisch. Hier führt die Probedestillation von 100 Teilen des Rohproduktes zu folgenden Werten:

Im Destillat (95 Teile) befinden sich
82,5% cis-2,6-Dimethylmorpholin
14,5% trans-2,6-Dimethylmorpholin.
Der Destillationsrückstand beträgt 4%.
Aus den angegebenen Daten errechnet sich eine Selektivität von 94%.

**Patentansprüche**

1. Verfahren zur Herstellung von cis-2,6-Dimethylmorpholin durch Isomerisierung von trans-2,6-Dimethylmorpholin, *dadurch gekennzeichnet,* daß man trans-2,6-Dimethylmorpholin in Gegenwart von Wasserstoff und in Gegenwart eines Katalysators, der ein oder mehrere Metalle der Gruppe VIII oder der Gruppe Ib des Periodensystems der Elemente oder deren Mischung enthält, bei Temperaturen zwischen 120 und 280°C, insbesondere zwischen 150 und 250°C und Drucken zwischen 1 und 200 bar umsetzt.

2. Verfahren gemäß Anspruch 1, *dadurch ge-*

*kennzeichnet,* daß der Katalysator zusätzlich zwischen 0,2 und 20 Gew.% eines Oxides oder eines Gemisches von Oxiden der seltenen Erdmetalle enthält.

3. Verfahren gemäß Anspruch 1, *dadurch gekennzeichnet,* daß der Katalysator in reiner Form oder als Mischkatalysator vorliegt.

4. Verfahren gemäß dem Anspruch 1, *dadurch gekennzeichnet,* daß der Katalysator einen inerten Träger enthält.

5. Verfahren gemäß dem Anspruch 1, *dadurch gekennzeichnet,* daß der Katalysator Palladium enthält.

**Claims**

1. A process for the preparation of cis-2,6-dimethylmorpholine by isomerizing trans-2,6-dimethylmorpholine, wherein trans-2,6-dimethylmorpholine is isomerized in the presence of hydrogen and of a catalyst which contains one or more metals of group VIII or group Ib of the periodic table of the elements, or contains a mixture of these, at from 120 to 280°C, especially from 150 to 250°C, under a pressure of from 1 to 200 bar.

2. A process as claimed in claim 1, wherein the catalyst additionally contains from 0.2 to 20 wt% of an oxide or of a mixture of oxides of the rare earth metals.

3. A process as claimed in claim 1, wherein the catalyst is in pure form or is present as a catalyst mixture.

4. A process as claimed in claim 1, wherein the catalyst contains an inert carrier.

5. A process as claimed in claim 1, wherein the catalyst contains palladium.

**Revendications**

1. Procédé de préparation de cis-2,6-diméthylmorpholine par isomérisation de trans-2,6-diméthylmorpholine, caractérisé par le fait que l'on fait réagir de la trans-2,6-diméthylmorpholine en présence d'eau et en présence d'un catalyseur qui contient un ou plusieurs métaux du groupe VIII ou de groupe Ib du système périodique ou un de leurs mélanges, à des températures comprises entre 120 et 280°C, en particulier entre 150 et 250°C, et sous des pressions comprises entre 1 et 200 bars.

2. Procédé selon la revendication 1, caractérisé par le fait que le catalyseur contient en outre entre 0,2 et 20% en poids d'un oxyde ou d'un mélange d'oxydes de terres rares.

3. Procédé selon la revendication 1, caractérisé par le fait que le catalyseur est sous forme pure ou sous forme de catalyseur mixte.

4. Procédé selon la revendication 1, caractérisé par le fait que le catalyseur contient un support inerte.

5. Procédé selon la revendication 1, caractérisé par le fait que le catalyseur contient du palladium.